# EUROPÄISCHE PATENTSCHRIFT

(11) **EP 0 829 243 B1**
(45) Veröffentlichungstag und Bekanntmachung des Hinweises auf die Patenterteilung: **24.07.2002**
(21) Anmeldenummer: 97114285.6
(22) Anmeldetag: 19.08.1997
(51) Int. Cl.: A61F 2/30, A61F 2/38

(54) **Gelenkendoprothese**
Joint endoprosthesis
Endoprothèse d'articulation

(30) Priorität: 12.09.1996 DE 29615920 U
(43) Veröffentlichungstag der Anmeldung: 18.03.1998
(73) Patentinhaber: Waldemar Link (GmbH & Co.), 22339 Hamburg (DE)
(72) Erfinder: Link, Helmut D., 22397 Hamburg (DE)
(74) Vertreter: Glawe, Delfs, Moll & Partner

(56) Entgegenhaltungen:
- EP-A- 0 786 238
- FR-A- 2 743 716
- US-A- 4 085 466
- US-A- 4 166 292
- US-A- 4 207 627
- US-A- 4 479 271
- US-A- 4 997 445
- US-A- 5 236 462

## Beschreibung

Die Erfindung betrifft eine Gelenkendoprothese mit einem aus gleitgünstigem Polymermaterial wie Polyethylen bestehenden Prothesenteil, der auf einer Seite eine Gleitfläche bildet und auf der anderen Seite zur Verbindung mit dem Knochen ohne biegefeste Zwischenlage eingerichtet ist, beispielsweise das Tibiaplateau einer Kniegelenkprothese.

Gleitgünstiges Polymermaterial wie hochdichtes Polyethylen hat einen niedrigen Elastizitätsmodul und neigt daher zur Durchbiegung unter örtlicher Belastung. Es wird deshalb in der Regel unter Verwendung einer biegefesten, meist metallischen Stützplatte implantiert, auf der das Plateau mit seiner gesamten Unterfläche aufliegt (US-A 4,997,445, US-A 5,236,462, US-A 4,207,627) oder mit einer anderen steifen Platte verbunden (EP-A 68 18 45). Wenn man es ohne eine solche starre Unterstützung einsetzt (DE-AS 19 64 781), muß es eine unerwünscht große Dicke haben. Diese Grundsätze gelten schon dann, wenn das Plateau bzw. seine Unterstützungsplatte durch Knochenzement mit dem Knochen verbunden wird. Erst recht gelten sie bei zementfreier Implantation, bei der eine Relativbewegung zwischen der Oberfläche des Implantats und dem Knochen vermieden werden muß. Erwünscht wäre im Hinblick auf die Vereinfachung des Prothesenaufbaus und die Verminderung der für das Plateau einschließlich seiner Unterstützung vorzusehenden Einbauhöhe ein Plateau, das trotz des geringen E-Moduls gleitgünstiger Polymerwerkstoffe eine hohe Biegefestigkeit aufweist. Bekannt ist es (DD-A 227 328), ein Tibiaplateau durch isotrope Einmischung von verfestigenden Fasern in das Polyethylenmaterial zu verstärken. Da diese die Homogenität des Materials in der Gleitfläche vermindern und somit die Gleit- und Verschleißeigenschaften beeinträchtigen, hat sich diese Maßnahme nicht bewährt. Das gilt auch für den Schichtaufbau eines Plateaus aus orientierten Polymerfasern (DE-A 40 06 714). Deshalb hat man versucht, eine Verstärkungsschicht in einem solchen Abstand von der Gleitoberfläche in eine Hüftpfanne einzubetten (DE-A 38 38 568), wie es der in der Praxis maximal zu erwartenden Verschleißdicke entspricht, so daß die Gesamtdicke des Implantats sich entsprechend vergrößert.

Bekannt sind auch Knieprothesen, bei denen die Gelenkfläche von einem weichelastischen Werkstoff ohne eigene Biegefestigkeit gebildet ist; dabei ist eine biegefeste Unterlage unabdingbar (US-A 4,085,466). Damit das weichelastische Material unter Belastung seine Form nicht verliert, wird es durch einen zugfesten Ring eingegrenzt. Ein solcher zugfester Ring ist auch erforderlich, wenn als Material pyrolytischer Kohlenstoff verwendet wird (US-A 4,166,292), weil dieser gegenüber Zugspannungen empfindlich ist, die sich dann ergeben können, wenn der Prothesenteil nicht durch eine Ringeinfassung, die infolge hohen Elastizitätsmoduls praktisch starr wirkt, an seitlicher Dehnung unter axialer Belastung gehindert wird. Die Biegefestigkeit des Implantats wird in keinem dieser beiden Fälle durch die Einschließung in einen Ring vergrößert.

Aus dem Stand der Technik nach Artikel 54(3) EPÜ ist eine Knieprothese bekannt (EP-A-786 283), bei der das aus Kunststoff bestehende Plateau zur Verbindung mit dem Knochen und zur Verstärkung einen Metallring an seiner Unterseite aufweist, der über eine Rastverbindung mit elastisch nachgiebigen Rastelementen mit dem Plateau verbunden ist.

Der Erfindung liegt die Aufgabe zugrunde, bei einer Gelenkendoprothese der eingangs genannte Art eine geringere Plateaudicke zu ermöglichen. Die erfindungsgemäße Lösung besteht in den Merkmalen der nebengeordneten Ansprüche 1 und 2.

Die Erfindung beruht auf der Erkenntnis, daß es für die Verringerung der Biegeneigung des Implantats nicht unbedingt erforderlich ist, die Verstärkung unmittelbar unterhalb des Belastungsorts vorzusehen. Wenn sie diesen in einer der Gleitfläche fernen Schicht zugfest und mit einem im Vergleich dazu höheren Elastizitätsmodul umgibt und ihm nahe benachbart ist, wird die Gesamtverbiegung des Implantats stark vermindert. Wie ohne weiteres einzusehen ist, kann der äußere, mit dem Verstärkungselement versehene Teil des Implantats der Biegebeanspruchung kaum nachgeben. Er verhält sich daher biegesteif. Da der zentrale, nicht unmittelbar mit einem Verstärkungselement versehene Bereich in dem äußeren, verstärkten Bereich sozusagen eingespannt ist, wird auch seine Biegefähigkeit verringert. Für eine gegebene Dicke ist die Gesamtverformung des Implantats unter einer gegebenen Last jedenfalls wesentlich geringer als bei vorbekannten, unverstärkten Implantaten. Gegenüber bekannten Implantaten, die eine Verstärkungsschicht enthalten, kann die Dicke beträchtlich reduziert werden, weil im zentralen, dem Verschleiß ausgesetzten Belastungsbereich eine Verschleißstrecke zur Verfügung steht, die durch das Verstärkungselement nicht reduziert ist. Die Erfindung führt somit zu dem Vorteil, daß Implantate vergleichsweise hohen Biegewiderstands bei geringer Dicke zur Verfügung gestellt werden können.

Die erforderliche Schubfestigkeit der Verbindung des Verstärkungselements mit den übrigen Implantatmaterial kann einerseits dadurch bewirkt werden, daß das Verstärkungselement allseits von dem übrigen Implantatmaterial umschlossen ist (Anspruch 1). Ohne eine solche Umschließung kann eine hinreichende solche Schubfestigkeit durch eine dauerhafte Verbindung des Verstärkungselements mit dem plattenförmigen Prothesenteil erzielt werden, die mindestens teilweise eine Klebeverbindung ist (Anspruch 2).

Vorzugsweise ist das Verstärkungselement in den plattenförmigen Prothesenteil eingebettet. Dies soll besagen, daß es diesen nicht nur an den Seiten ringförmig umgibt. Es kann daher näher am zentralen, belasteten und verschleißgefährdeten Bereich angeordnet werden, so daß es seine die Biegesteifigkeit erhöhende Wirkung nahe diesem Bereich entfalten kann.

Wichtig ist, daß es der radialen Ausdehnung der bei Biegung einer Dehnung unterworfenen unteren Schicht des Plateaus entgegenwirkt. Aus diesem Grunde wird man es häufig für zweckmäßig halten, das Verstärkungselement ausschließlich in der unteren Hälfte oder gar im unteren Drittel oder unteren Viertel der Plateaudicke anzuordnen. Der Begriff "unten" bedeutet: von der Gleitfläche abgelegen.

Um den Hauptbelastungsbereich umgehen zu können, ist die Verstärkung vorzugsweise ringförmig gestaltet. Kreisringform bildet die geometrisch besten Voraussetzungen für die Kraftaufnahme, ist aber nicht erforderlich. In der Regel genügt beispielsweise eine ovale oder D-förmige Gestalt.

Das Merkmal, daß das Implantat plattenförmig sein soll, besagt, daß das Verstärkungselement sich in derselben Ebene befinden kann, in welcher sich innerhalb des hauptsächlich beanspruchten Bereichs Zugspannungen entwickeln können. Bei einem hohlsphärischen Implantat, wie beispielsweise einer Hüftpfanne, ist diese Bedingung nicht erfüllt, weil eine dehnende Deformation der von der Gleitfläche fernen Schicht des Implantats im Hauptbelastungsbereich nicht durch eine weiter außen befindliche, in einer anderen Ebene liegende Verstärkung wesentlich behindert werden kann. Das Hauptanwendungsgebiet der Erfindung betrifft daher Tibiaplateaus von Kniegelenkprothesen. Auf die Gestalt des Implantats auf seiner Gleitflächenseite kommt es für den Begriff "plattenförmig" nicht an.

Die Erfindung wird im folgenden näher unter Bezugnahme auf die Zeichnung erläutert, die vorteilhafte Ausführungsbeispiele schematisch veranschaulicht. Darin zeigen:
- Fig.1: eine schematische Darstellung eines Tibiaplateaus mit der zugeordneten femoralen Gleitfläche im Sagittalschnitt,
- Fig.2: eine Draufsicht auf das Tibiaplateau gemäß Fig.1 und
- Fig.3 bis 6: Sagittalschnitte durch alternative Ausführungsformen des Tibiaplateaus.

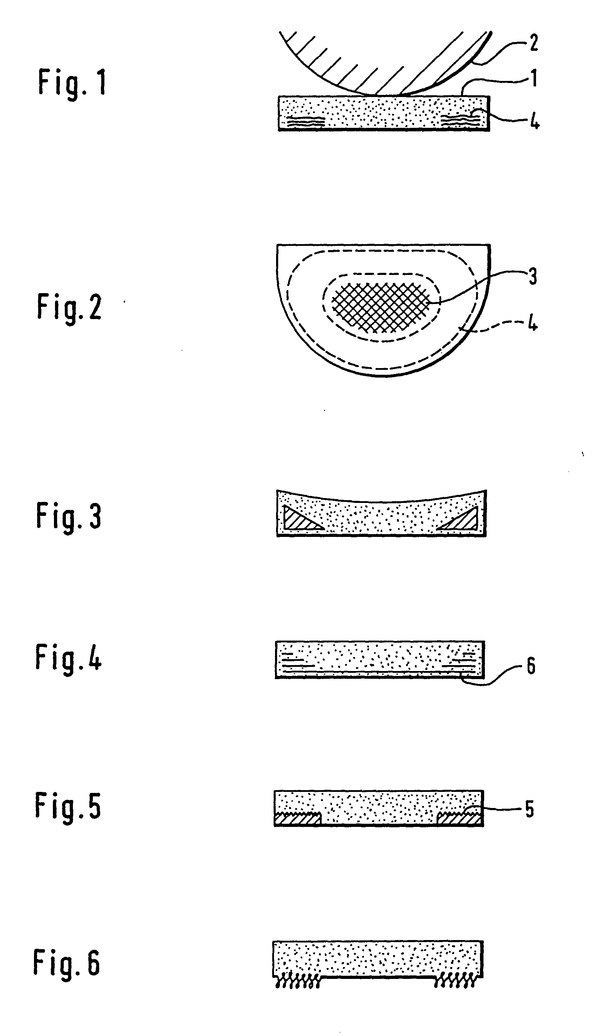

Im Beispiel der Fig.1 ist das Plateau eine flache Scheibe mit ebener, oberseitiger Gleitfläche 1 und etwa halbkreisförmigem Umriß, die mit einer gekrümmten femoralen Gleitfläche 2 zusammenwirkt. Die oberseitige Gleitfläche 1 des Plateaus kann auch gemuldet sein, wie dies in Fig.3 angedeutet ist, bis hin zur teilweisen Kongruenz der Gleitflächen 1 und 2. In der Regel wird aber die plateauseitige Gleitfläche 1 flach sein oder einen wesentlich größeren Krümmungsradius als die damit zusammenwirkende Gleitfläche 2 haben. Daraus ergibt sich, daß die Beanspruchung des Plateaus hauptsächlich innerhalb eines begrenzten Bereichs stattfindet, der oben als Hauptbelastungsbereich oder Hauptverschleißbereich bezeichnet wurde und der in Fig.2 kreuzschraffiert beispielsweise angedeutet ist. Die Stelle, an der die Gegengleitfläche 2 die Gleitfläche 1 des Plateaus berührt, kann je nach den individuellen Voraussetzungen und der zufälligen Relativstellung der Gelenkkomponenten zueinander an unterschiedlicher Stelle innerhalb des Hauptbelastungsbereichs 3 liegen. Bei der Konstruktion der Prothese wird der Hauptbelastungsbereich als derjenige angesehen, in welchem statistisch die Belastung meistens auftritt.

Aufgrund dieser stellenweise stärkeren Belastung des Plateaus ist dieses einer Biegebeanspruchung ausgesetzt, die um so stärker ist, je nachgiebiger die Unterstützungsfläche ist, die von dem natürlichen Knochengewebe gebildet ist, das nach Resektion unter der Plateaumitte in vielen Fällen eine spongiöse Struktur hat und daher vergleichsweise nachgiebig ist. Das gilt nicht nur bei zementfreier Implantation, sondern auch bei Verankerung des Plateaus mittels Zement. Diese Biegebeanspruchung führt im unteren Teil des Plateaus in der Nähe der Beanspruchungsstelle zu einer Zugspannung, die eine Dehnung des Plateaus im unteren Teil zur Folge hat. Diese Dehnung kann je nach den Fließeigenschaften des Materials auch zu dauernder Deformation führen, wenn nicht geeignete Vorkehrungen getroffen sind. Diese Vorkehrungen bestehen erfindungsgemäß aus einem ringförmig hauptsächlich im unteren Bereich des Plateaus angeordneten Verstärkungselement 4, das den Hauptbelastungsbereich 3 eng umschließt. Es ist in Umfangsrichtung zugfest und hat einen höheren Elastizitätsmodul als das sonstige, im Schnitt punktiert angedeutete Plateaumaterial und wirkt dadurch der aufgrund der Biegebeanspruchung des Plateauteils in seinem unteren Teil zu erwartenden Dehnung entgegen. Es kann diese Dehnung nicht vollständig verhindern. Dies ist aber auch nicht erforderlich, solange sie auf einen so niedrigen Betrag begrenzt wird, daß das darunter befindliche, nachgiebige Knochengewebe der Dehnungsbewegung folgen kann und somit keine oder keine wesentliche Relativbewegung zwischen der Plateauunterfläche und dem Knochengewebe, stattfindet.

Dadurch, daß das Verstärkungselement den Hauptbelastungsbereich frei läßt, steht dort eine unverminderte Verschleißreserve zur Verfügung. Die Anwesenheit des Verstärkungselements in dem Plateau zwingt daher nicht zu einer stärkeren Dickenbemessung; vielmehr kann die Dickenbemessung frei nach anderen Gesichtspunkten erfolgen.

Die Querschnittsform des Verstärkungselements in Fig.1 ist flach scheibenförmig, während in Fig.3 eine Querschnittsgestalt gezeigt ist, bei der die Dicke des Verstärkungselements zum Hauptbelastungsbereich 3 hin sich vermindert und seine obere Begrenzungsfläche nach unten abfällt. Dadurch kann das Verstärkungselement näher an den Hauptbelastungsbereich 3 herangeführt werden oder sogar mit seinem inneren Rand in ihn hineinreichen. Eine Verjüngung des Verstärkungselements nach innen hin kann auch dazu eingesetzt werden, die Nachgiebigkeit des Plateaus örtlich unterschiedlich einzustellen, um sie nach innen hin anwachsen zu lassen. Die örtliche Nachgiebigkeit des Implantats kann dadurch derjenigen des Knochens in gewünschter Weise angepaßt werden.

Dieser Gedanke ist in Fig.4 weitergeführt, die ein Ausführungsbeispiel zeigt, in welchem eine dünne Schicht 6 des Verstärkungselements unterhalb des Hauptbelastungsbereichs 3 hindurchgeführt ist, während der Hauptquerschnitt des Verstärkungselements sich, wie oben erläutert, ringförmig außerhalb des Hauptbelastungsbereichs 3 befindet. Diese dünne und sich auf die Dicke des Plateaus praktisch nicht auswirkende Schicht 6 dient hauptsächlich dazu, die im unteren Bereich des Plateaus auftretende Zugspannung auf das ringförmig außerhalb des Hauptbelastungsbereichs (3) befindliche Verstärkungselement (4) zu übertragen.

Während die Ausführungsbeispiele der Fig.1 bis 4 die allseitige Einschließung des Verstärkungselements in das Plateaumaterial zeigen, ist in Fig.5 vorausgesetzt, daß das Plateaumaterial innerhalb eines ringförmigen Randbereichs einen Rezeß aufweist, in den das Verstärkungselement formgleich und schubfest eingesetzt ist. Die Schubfestigkeit ist in der Zeichnung dadurch angedeutet, daß die zusammenwirkenden Flächen 5 des Rezesses und des Verstärkungselements rauh ineinandergreifend, beispielsweise mit zirkulären Rippen und Nuten, ausgebildet sind. Es ist eine dauerhafte Verbindung vorgesehen, deren Schubfestigkeit nicht oder nicht nur durch die Rauhigkeit, sondern auch durch eine Klebeverbindung vermittelt wird. Beispielsweise kann das Verstärkungselement von einem Faserstrang gebildet sein, der mit einem geeigneten, zunächst bildsamen Kunststoff getränkt ist und das in den Rezeß bis zur Erhärtung eingepreßt wird, wobei das Kunstharz eine chemische oder physikalische Bindung mit der Oberfläche des Plateaumaterials eingeht und sich in dessen Rauhigkeit einbettet. Es kann sich auch um einen Metallring handeln.

Schließlich ist in Fig.6 ein Ausführungsbeispiel dargestellt, das schematisch andeutet, daß das Verstärkungselement nicht nur in das Plateau eingebettet oder damit innig verbunden ist, sondern auch unter die Unterfläche des Plateaus vorragende Vorsprünge (Schlaufen, Häkchen, Porosität etc.) bildet, die zur zementierten oder zementfreien Verbindung mit dem Knochen geeignet sind.

In den anderen Ausführungsbeispielen wurde davon abgesehen, Verankerungselemente darzustellen und zu erläutern, die die Verbindung zwischen dem Plateau und dem zugehörigen Knochen vermitteln. Es versteht sich, daß in der Regel solche Verankerungsorgane in Form von Zapfen, Vorsprüngen, poröser Beschichtung oder dergleichen vorgesehen werden.

Das Verstärkungselement kann aus jedem Material bestehen, das die erstrebte Zugfestigkeit zu liefern vermag, beispielsweise Fasern, Fäden, Drähte, Ringe aus Polymermaterial oder Metall. Diese können unmittelbar in das Plateaumaterial eingebettet oder damit verbunden sein. Sie können auch im Falle von Fasern oder Fäden mit einer Kunstharzimprägnierung versehen und dadurch zu einem einheitlichen Gebilde vereinigt sein, das wiederum mit dem Material des Plateaus verbunden ist. Das Verstärkungselement ist vorzugsweise flexibel, kann aber auch starr sein.

Wenn im vorliegenden Zusammenhang von einem "unteren" Bereich des Plateaus gesprochen wird, so ist damit der der Gleitfläche 1 abgewandte Bereich gemeint.

## Patentansprüche

1. Gelenkendoprothese mit einem auf einer Seite eine Gleitfläche (1) bildenden und an der anderen Seite zur Verbindung mit dem Knochen ohne biegefeste Zwischenlage eingerichteten, im wesentlichen plattenförmigen Prothesenteil aus gleitgünstigem Polymermaterial wie Polyethylen, der einen zentral gelegenen, überwiegend der Belastung und dem Verschleiß ausgesetzten Bereich aufweist, und mit einem zumindest teilweise in dem von der Gleitfläche (1) abgewandten Dickendrittel enthaltenen zugfesten Verstärkungselement (4), das einen höheren Elastizitätsmodul als der plattenförmige Prothesenteil aufweist, im wesentlichen außerhalb des Hauptbelastungs- und -verschleißbereichs (3) angeordnet ist und diesen umschließt, und das vollständig von dem übrigen Material des Prothesenteils umschlossen ist.

2. Gelenkendoprothese mit einem auf einer Seite eine Gleitfläche (1) bildenden und an der anderen Seite zur Verbindung mit dem Knochen ohne biegefeste Zwischenlage eingerichteten, im wesentlichen plattenförmigen Prothesenteil aus gleitgünstigem Polymermaterial wie Polyethylen, der einen zentral gelegenen, überwiegend der Belastung und dem Verschleiß ausgesetzten Bereich aufweist, und mit einem zumindest teilweise in dem von der Gleitfläche (1) abgewandten Dickendrittel enthaltenen zugfesten Verstärkungselement (4), das einen höheren Elastizitätsmodul als der plattenförmige Prothesenteil aufweist, im wesentlichen außerhalb des Hauptbelastungs- und -verschleißbereichs (3) angeordnet ist und diesen umschließt, und das mit dem plattenförmigen Prothesenteil mindestens teilweise durch eine Klebeverbindung verbunden ist.

3. Gelenkendoprothese nach Anspruch 2, **dadurch gekennzeichnet, daß** das Verstärkungselement (4) in den Prothesenteil eingebettet ist.

4. Gelenkendoprothese nach Anspruch 3, **dadurch gekennzeichnet, daß** das Verstärkungselement (4) vollständig von dem übrigen Material des Prothesenteils umschlossen ist.

## Claims

1. A joint endoprosthesis with a substantially plate-like prosthesis part of polymeric material having a low coefficient of friction, such as polyethylene, which on one side forms a sliding surface (1) and on the other side is arranged for connection with the bone without a bending-resistant intermediate layer, and which has a centrally positioned region predominantly exposed to load and wear, and with a tension-resistant reinforcing element (4) which is contained at least partly in a third part of the thickness remote from the sliding surface (1), has a higher modulus of elasticity than the plate-like prosthesis part and is arranged substantially outside the principal loading and wear zone (3) and surrounds it, and which is entirely surrounded by the remaining material of the prosthesis part.

2. A joint endoprosthesis with a substantially plate-like prosthesis part of polymeric material having a low coefficient of friction, such as polyethylene, which on one side forms a sliding surface (1) and on the other side is arranged for connection with the bone without a bending-resistant intermediate layer, and which has a centrally positioned region predominantly exposed to load and wear, and with a tension-resistant reinforcing element (4) which is contained at least partly in a third part of the thickness remote from the sliding surface (1), has a higher modulus of elasticity than the plate-like prosthesis part and is arranged substantially outside the principal loading and wear zone (3) and surrounds it, and which is connected with the plate-like prosthesis part at least partly by a bonded joint.

3. A joint endoprosthesis according to Claim 2, **characterised in that** the reinforcing element (4) is embedded in the prosthesis part.

4. A joint endoprosthesis according to Claim 3, **characterised in that** the reinforcing element (4) is entirely surrounded by the remaining material of the prosthesis part.

## Revendications

1. Endoprothèse d'articulation comportant une partie de prothèse sensiblement en forme de plaque en une matière polymère favorisant le glissement telle que du polyéthylène, formant une surface de glissement (1) sur un côté et aménagée sur l'autre côté en vue de la liaison avec l'os sans couche intermédiaire résistante à la flexion, laquelle partie de prothèse comporte une zone située au centre, principalement exposée à la charge et à l'usure, et comportant un élément de renfort (4) résistant à la traction contenu au moins en partie dans le tiers de l'épaisseur tourné à l'opposé de la surface de glissement (1), qui présente un module d'élasticité supérieur à la partie de prothèse en forme de plaque, est disposé sensiblement à l'extérieur de la zone de charge principale et d'usure (3), et entoure celle-ci, et qui est entièrement enfermé par le reste de la matière de la partie de prothèse.

2. Endoprothèse d'articulation comportant une partie de prothèse sensiblement en forme de plaque en une matière polymère favorisant le glissement telle que du polyéthylène, formant une surface de glissement (1) sur un côté et aménagée sur l'autre côté en vue de la liaison avec l'os sans couche intermédiaire résistante à la flexion, laquelle partie de prothèse comporte une zone située au centre, principalement exposée à la charge et à l'usure, et comportant un élément de renfort (4) résistant à la traction, contenu au moins en partie dans le tiers de l'épaisseur tourné à l'opposé de la surface de glissement (1), qui présente un module d'élasticité supérieur à la partie de prothèse en forme de plaque, est disposé sensiblement à l'extérieur de la zone de charge principale et d'usure (3) et entoure celle-ci et qui est relié à la partie de prothèse en forme de plaque au moins partiellement par une liaison collée.

3. Endoprothèse d'articulation selon la revendication 2, **caractérisée en ce que** l'élément de renfort (4) est noyé dans la partie de prothèse.

4. Endoprothèse d'articulation selon la revendication 3, **caractérisée en ce que** l'élément de renfort (4) est entièrement enfermé par le reste de la matière de la partie de prothèse.
